# EUROPEAN PATENT APPLICATION

(11) **EP 4 309 684 A1**
(43) Date of publication of application: **24.01.2024**
(21) Application number: 22306073.2
(22) Date of filing: 20.07.2022
(51) Int. Cl.: A61L 27/36, A61L 27/44, A61L 27/52, B33Y 10/00, B33Y 80/00

(54) **HYDROGEL WITH A HYDROPHILIC THICKENING POLYMER AND PARTICLES OF EGGSHELL MEMBRANE, AND ITS BIOPRINTED PRODUCTS**

(71) Applicant: Université Côte d'Azur, 06100 Nice (FR); Centre Hospitalier Universitaire de Nice, 06000 Nice (FR)
(72) Inventor: PRATE, Frédéric, 06100 NICE (FR); ZENERINO, Arnaud, 06440 BLAUSASC (FR)
(74) Representative: Lavoix

(57) **Abstract**

The present invention concerns a composition which is a hydrogel comprising, in an aqueous medium:
- at least one hydrophilic thickening polymer, and
- particles of eggshell membrane, in an amount of less than 5% by weight of the total weight of the composition, wherein said particles are rod-shaped, needle-shaped or fibrous, having a specific particle size.
It also relates to a bioink comprising said composition, to a method for manufacturing a 2D or 3D product using said hydrogel, to a kit comprising said hydrogel or bioink, and to uses thereof.

## Description

The present invention concerns a composition which is a hydrogel comprising, in an aqueous medium:
- at least one hydrophilic thickening polymer, and
- particles of eggshell membrane, in an amount of less than 5% by weight of the total weight of the composition, wherein said particles are rod-shaped, needle-shaped or fibrous, having a specific particle size.
It also relates to a bioink comprising said composition, to a method for manufacturing a 2D or 3D product using said hydrogel, to a kit comprising said hydrogel or bioink, and to uses thereof.

Three-dimensional (3D) printing is a growing field in regenerative medicine and represents a valuable tool for the construction of complex 3D structures.

In literature, two approaches involving 3D printing are described: first, the use of 3D printing to build an acellular scaffold which is seeded with cells after fabrication (Top-Down approach), and secondly, the creation of tissue constructs by directly printing a biological ink or bioink composed of a mixture of cells and biomaterial (Bottom-Up approach). This latter method can be performed through several techniques, such as inkjet, laser and extrusion-based bioprintings.

Regarding these different methods, extrusion-based bioprinting (EBB) is part of the most widespread tools used in additive manufacturing, mainly due to its capabilities for building large volume constructs such as tissue or organ equivalents.

Among all types of bioinks, hydrogels seem to be a promising biomaterial as they are easy to handle, affordable, and can mimic extracellular matrix. Hydrogels are mainly composed of water entrapped in a 3D network of molecules, which can be covalently (chemically) or physically (ionic, hydrogen bonds or hydrophobic effect) crosslinked.

Initial approaches of extrusion-based bioprinting used synthetic polymers such as polyethylene glycol (PEG) as scaffold materials but they suffer from poor biomimicry as their synthetic nature does not provide them a biological activity for their use in cell culture. As an alternative, natural polymers derived from fibrous proteins or peptides have been developed for their original biological activities.

Among natural polymers, alginate, chitosan, collagen and gelatin are actually the most studied despite their weaknesses. Indeed, these polymers encounter several issues such as low mechanical properties or the need of an external crosslinker to form a solid-like structure. Moreover, a prerequisite for a bioink is its capacity to be formulated in a cell culture medium, as for cell viability.

There is therefore a need for a hydrogel that does not only provide a biological activity for its use in cell culture but also high mechanical properties.

Inventors have thus successfully developed a bioink formulation that fulfils both the need of a biological activity and high mechanical properties.

As shown in the examples, inventors have shown that a hydrogel comprising at least one hydrophilic thickening polymer, and specific particles of eggshell membrane in an aqueous medium, is a promising bioink.

The present invention thus relates to a composition which is a hydrogel comprising, in an aqueous medium:
- at least one hydrophilic thickening polymer, and
- particles of eggshell membrane, in an amount of less than 5% by weight of the total weight of the composition, wherein said particles are rod-shaped, needle-shaped or fibrous, having a particle size equal to or less than 50 µm.

Said composition is a composition according to the invention, or a hydrogel. Both terms are used interchangeably.

The composition of the invention (hydrogel) is biocompatible in order to become a bioink. By "bioink", it is meant a composition made up of at least a hydrogel and isolated cells. Bioinks are materials that mimic an extracellular matrix environment to support the adhesion, proliferation and differentiation of living cells. Depending on the bioprinting technology that is used, the bioink may be obtained by different processes. For instance, micro-droplets of bioink containing both a hydrogel and cells may be projected on a support. As an alternative, the bioprinter may work with two printheads, one depositing the hydrogel and the other the cells. The cells are pushed through a micro-syringe and deposited using a needle. The layers are alternately deposited, a hydrogel layer followed by a layer of cells. The hydrogel is used to structure the assembly of cell layers, similar to scaffolding. Thus, when the composition of the invention (hydrogel) further comprises cells, it is a bioink.

The invention also relates to a kit comprising:
- a first composition, wherein said first composition is according to the invention (i.e. hydrogel or bioink as described); and
- a second composition which comprises, in a compatible medium, at least one crosslinking agent; and
- optionally a third composition comprising at least one compound chosen from cells, coloring agents, pharmacologic agents, differentiation factors, growth factors, biological markers and their mixtures.

The invention also relates to the use of a composition or bioink according to the invention, or of a kit of the invention, as an *in vitro* research model, or for obtaining a 2D (single layer) or a 3D product, i.e. such as an organoid, an organ or a biological tissue. A 2D product may be of interest in the case of a product comprising a monolayer of cells of a given predetermined structure.

The invention also relates to a method for manufacturing a 2D or 3D product, comprising the following steps:
(a) preparing a composition or bioink of the invention, preferably by heating said composition to a temperature comprised between 35°C and 40°C and centrifugating it;
(b) introducing the composition obtained at the end of step (a) into at least one first cartridge of a bioprinter, said bioprinter comprising at least one printbed, at least one printhead linked to said first cartridge and having a nozzle, preferably two printheads each having a nozzle, and optionally a UV curing system; and
(c) manufacturing the 2D or 3D product in the bioprinter.
The manufacturing step (c) is preferably performed at a printhead temperature comprised between 35°C and 40°C, a printbed temperature comprised between 3°C and 12°C, a printing pressure comprised between 25 and 35 KPa, a nozzle speed comprised between 5 and 15 mm/s, and a nozzle inner diameter comprised between 0.23 to 0.45 mm.

The invention also relates to an isolated 2D (single layer) or 3D product, i.e. an organoid, an organ or a biological tissue, which is obtainable by said method.

### Composition (or hydrogel)

The composition of the invention is a hydrogel comprising, in an aqueous medium:
- at least one hydrophilic thickening polymer, and
- particles of eggshell membrane, in an amount of less than 5% by weight of the total weight of the composition, wherein said particles are rod-shaped, needle-shaped or fibrous, having a particle size equal to or less than 50 µm.

All the particles of eggshell membrane (D100) have a particle size equal to or less than 50 µm.

Preferably, they have a D99.5 particle size equal to or less than 40 µm. More preferably, they have a D90 particle size equal to or less than 24 µm.

### Aqueous medium

The composition according to the invention comprises an aqueous medium.

Said aqueous medium preferably comprises water. Said water may be deionized.

More preferably, the aqueous medium only consists in water.

Preferably, the aqueous medium is present in an amount of at least 90% by weight of the total weight of the composition, preferably at least 92% by weight, preferably at least 94% by weight.

Preferably, water is present in an amount of at least 90% by weight of the total weight of the composition, preferably at least 92% by weight, preferably at least 94% by weight.

### Hydrophilic thickeners

The composition according to the invention comprises at least one hydrophilic thickening polymer (also called aqueous phase thickening polymer).

The thickening agents are also referred to interchangeably herein as thickeners or rheology modifiers. Thickening agents are generally used to modify the viscosity or rheology of compositions.

Non-limiting examples of thickening agents that may be used according to various embodiments of the disclosure include those conventionally used in cosmetics, such as polymers of natural origin and synthetic polymers. For example, nonionic, anionic, cationic, amphiphilic and amphoteric polymers, and other known rheology modifiers, such as cellulose-based thickeners, may be chosen.

The thickening agents are hydrophilic thickeners. As used herein, the term "hydrophilic thickener" is meant to indicate that the thickening agent is soluble or dispersible in water.

More particularly, this thickening polymer is chosen from:
- alginates and carrageenans. The alginate may be alginic acid, a divalent metal ion alginate, a divalent metal ion alginate and/or a monovalent metal ion alginate, in particular Ca2⁺ and/or Na⁺ alginate. The alginate will typically be a polymer, for example of at least 35kDa, or a plurality of polymers of different sizes;
- polymers of animal origin (such as proteins), possibly modified, such as gelatin or collagen;
- anionic, cationic, amphoteric or non-ionic chitin or chitosan polymers;
- cellulose polymers, in particular chosen from hydroxyethylcellulose, hydroxypropylcellulose, hydroxymethylcellulose, ethylhydroxyethylcellulose, carboxymethylcellulose, and quaternized cellulose derivatives;
- vinyl polymers, such as polyvinylpyrrolidones, methylvinyl ether and malic anhydride, vinyl acetate and crotonic acid copolymer, vinylpyrrolidone and vinyl acetate copolymers; vinylpyrrolidone and caprolactam copolymers; polyvinyl alcohol;
- polymers of natural origin, possibly modified, such as galactomannans and derivatives thereof, such as Konjac gum, Gellan gum, Carob gum, Fenugrec gum, Karaya gum, Tragacanth gum, gum arabic, gum acacia, guar gum, hydroxypropylguar, hydroxypropylguar modified by sodium methylcarboxylate groups (Jaguar XC97-1, Rhodia), ammonia trimethyl hydroxypropyl guar chloride, xanthan gum and the derivatives of xanthan, agar and its derivatives such as agarose;
- mucopolysaccharides such as chondroitin sulfates or hyaluronic acid and its derivatives, such as sodium hyaluronate, sodium acetylated hyaluronate, dimethylsilanol hyaluronate, sodium stearoyl hyaluronate, potassium hyaluronate, propyleneglycol hyaluronate, sodium hyaluronate crosspolymer, methacrylated hyaluronic acid, hydroxypropyl trimonium hyaluronate, hydrolyzed hyaluronic acid, hydrolyzed sodium hyaluronate and zinc hydrolyzed hyaluronate;
- homo- or copolymers of acrylic or methacrylic acid or the salts thereof and the esters thereof and in particular the products sold under the names VERSICOL F or VERSICOL K by ALLIED COLLOID, UTRAHOLD 8 by CIBA-GEIGY, polyacrylic acids of the SYNTHALEN K type, and the salts, in particular sodium, of polyacrylic acid (with the INCI name of sodium acrylate copolymer) and more particularly a cross-linked sodium polyacrylate (with the INCI name of sodium acrylate copolymer (and) caprylic/capric triglyceride) sold under the name LUVIGEL EM,
- acrylic and acrylamide acid copolymers such as those sold in the form of their sodium salt under the names RETEN by HERCULES, sodium polymethacrylate sold under the name DARVAN N°7 by VANDERBILT, the sodium salts of polyhydroxycarboxylic acids sold under the name HYDAGEN F by HENKEL,
- polyacrylic acid/alkyl acrylates copolymers, preferably carboxyvinyl polymers modified or not, and notably sold under the INCI name carbomer. The acrylate/C10-C30-alkylacrylate (INCI name : Acrylates/C10-30 Alkyl acrylate Crosspolymer) copolymers are very particularly preferred such as the products sold by Lubrizol under the trade names PEMULEN TR1, PEMULEN TR2, CARBOPOL 1382, CARBOPOL EDT 2020 and more preferably PEMULEN TR2,
- homopolymers and copolymers with an acrylamido propane sulfonic acid base, such as for example:
   - polyacrylamidomethyl propane sulfonic acid partially neutralized with ammonia and highly cross-linked, sold in particular by CLARIANT, for example under the name HOSTACERIN AMPS,
   - copolymers of acrylamidomethyl / acrylamide propane sulfonic acid for example of the SEPIGEL or SIMULGEL type sold in particular by SEPPIC,
   - copolymers of acrylamidomethyl / methylacrylate propane sulfonic acid of polyoxyethylene alkyl (cross-linked or not) among others of the ARISTOFLEX HMS, ARISTOFLEX TAC type, sold by CLARIANT,
   - copolymers of acrylamidomethyl propane sulfonic acid and of hydroxyethyl acrylate, such as for example the acrylamidomethyl propane sulfonic acid / hydroxyethyl acrylate copolymer such as in particular the one used in the commercial product sold under the name SIMULGEL NS by SEPPIC, or the acrylamidomethyl propane sulfonic acid / hydroxyethyl acrylate copolymer such as in particular the one used in the commercial product sold under the name SEPINOV EMT 10 sold by SEPPIC (INCI name: HYDROXYETHYL ACRYLATE/SODIUM ACRYLOYLDIMETHYL TAURATE COPOLYMER);
   - and mixtures thereof.

Preferably, the hydrophilic thickening polymer is chosen from alginates and carrageenans; polymers of animal origin, possibly modified ; anionic, cationic, amphoteric or non-ionic chitin or chitosan polymers; cellulose polymers; vinyl polymers ; polymers of natural origin, possibly modified, such as galactomannans and derivatives thereof, xanthan gum and the derivatives of xanthan, agar and its derivatives; mucopolysaccharides ; homo- or copolymers of acrylic or methacrylic acid or the salts thereof and the esters thereof; acrylic and acrylamide acid copolymers; polyacrylic acid/alkyl acrylates copolymers; homopolymers and copolymers with an acrylamido propane sulfonic acid base ; and mixtures thereof.

More preferably, the hydrophilic thickening polymer is chosen from alginates and carrageenans; gelatin, collagen; anionic, cationic, amphoteric or non-ionic chitin or chitosan polymers; hydroxyethylcellulose, hydroxypropylcellulose, hydroxymethylcellulose, ethylhydroxyethylcellulose, carboxymethylcellulose, and quaternized cellulose derivatives; polyvinylpyrrolidones, methylvinyl ether and malic anhydride, vinyl acetate and crotonic acid copolymer, vinylpyrrolidone and vinyl acetate copolymers; vinylpyrrolidone and caprolactam copolymers; polyvinyl alcohol; Konjac gum, Gellan gum, Carob gum, Fenugrec gum, Karaya gum, Tragacanth gum, gum arabic, gum acacia, guar gum, hydroxypropylguar, hydroxypropylguar modified by sodium methylcarboxylate groups, ammonia trimethyl hydroxypropyl guar chloride, xanthan gum, agar and agarose; chondroitin sulfates, hyaluronic acid, sodium hyaluronate, sodium acetylated hyaluronate, dimethylsilanol hyaluronate, sodium stearoyl hyaluronate, potassium hyaluronate, propyleneglycol hyaluronate, sodium hyaluronate crosspolymer, methacrylated hyaluronic acid, hydroxypropyl trimonium hyaluronate, hydrolyzed hyaluronic acid, hydrolyzed sodium hyaluronate and zinc hydrolyzed hyaluronate; polyacrylic acids and the salts, in particular sodium, of polyacrylic acid and more particularly a cross-linked sodium polyacrylate ; sodium polymethacrylate, the sodium salts of polyhydroxycarboxylic acids ; carboxyvinyl polymers modified or not ; polyacrylamidomethyl propane sulfonic acid partially neutralized with ammonia and highly cross-linked ; copolymers of acrylamidomethyl / acrylamide propane sulfonic acid ; copolymers of acrylamidomethyl / methylacrylate propane sulfonic acid of polyoxyethylene alkyl (cross-linked or not) ; copolymers of acrylamidomethyl propane sulfonic acid and of hydroxyethyl acrylate ; and mixtures thereof.

According to a preferred embodiment of the invention, the composition (i.e. the hydrogel) comprises at least two hydrophilic thickening polymers.

Preferably the hydrophilic thickening polymers are at least alginate and gelatin.

Preferably, the amount of hydrophilic thickening polymer(s) (in active material) ranges from 0.01 to 20% by weight, relative to the total weight of the composition, preferably from 0.02 to 15% by weight, more preferably from 0.1 to 10% by weight, relative to the total weight of the composition.

### Particles of eggshell membrane

The composition according to the invention comprises particles of eggshell membrane.

According to the invention, particles of eggshell membrane are also called eggshell membrane (ESM) particles or ESM particles.

These particles of eggshell membrane are preferably present in an amount of 0.1% to 4% by weight, preferably from 0.25% to 2% by weight of the total weight of the composition.

The known particle size analysis methods are suitable for determining the median particle size, for example particle size measurement using light, for example light-scattering methods or turbidimetric methods, sedimentation methods, for example pipette analysis using an Andreassen pipette, sedimentation scales, photosedimentometers or sedimentation in a centrifugal force field, pulse methods, for example using a Coulter counter, or sorting by means of gravitational or centrifugal force. Those methods are described, inter alia, in Voigt, loc. cit., pages 64-79.

All the particles of eggshell membrane (D100) have a particle size equal to or less than 50 µm.

Preferably, they have a D99.5 particle size equal to or less than 40 µm. More preferably, they have a D90 particle size equal to or less than 24 µm.

The "DX value" stated in the present description corresponds to the size of particles defined such that X% of the volume of the particles have a size equal to or less than said DX value. Typically the particle size analysis is done by a laser diffraction method and meets the standards set forth in ISO 13320-1. The measurement can be done on a Malvern Mastersizer 2000. For example, a D90 value equal to or less than 24 µm corresponds to the size of particles defined such that 90% of the volume of the particles have a size equal to or less than 24 µm.

An ESM particle typically has one length dimension that is significantly greater than the others and so may be referred to as, for example, rod-shaped, needle-shaped or fibrous (rods, needles or fibres) and may be qualified as cylindrical or parallelepiped depending on the cross-sectional shape substantially perpendicular to the dimension of significantly greater length.

An ESM particle may have an aspect ratio between a first length dimension and a second length dimension arranged perpendicular thereto of at least 1.5 (first length dimension: second length dimension), preferably of at least 2, 3, 4, 5, 6, 7, 8, 9, 10, 12, 14, 16, 18, 20, 25, 30, 35, 40, 45, 50, 55, 60, 65, 70, 80, 90 or 100. The first length dimension is the longest length dimension in the particle and may be termed the longitudinal dimension L. The second length dimension may therefore be termed a lateral dimension I. The second length dimension is the longest lateral dimension or a mean average value of the lateral dimensions of the particle.

The ESM particles of the invention are rod-shaped, needle-shaped or fibrous. These terms stated in the present description mean that more than 80%, preferably more than 90%, of the particles are rod-shaped, needle-shaped or fibrous, which means a parallelepiped shape. The ESM particles of the invention have a particle size equal to or less than 50 µm.

ESM is the fibrous bilayer found in an egg between the albumen and the eggshell of avian eggs, such as the eggs of fowl (gamefowl/landfowl (Galliformes) and waterfowl (Anseriformes)) and poultry, in particular chicken, duck, goose, turkey, guineafowl, ostrich, pigeon, pheasant, partridge, grouse or gull. The eggs of *Gallus gallus domesticus,* the domestic chicken, are especially preferred. Either or both layers of the bilayer may be used in accordance with the invention.

Preferably the ESM particles of the invention are essentially free of other egg components (which may be considered "contaminating" substances vis-à-vis ESM), called non-ESM egg components, i.e. albumen, yolk, and/or egg shell (calcium carbonate). By "essentially free", it is meant that the ESM particles contain less than 5% by weight of the total weight of ESM particles, preferably less than 4%, 3%, 2%, 1%, 0.5%, 0.1%, 0.05% or 0.01% by weight, of non-ESM egg components.

Preferably the ESM particles comprise at least one cysteine-rich eggshell membrane protein (CREMP) and/or collagen X; preferably comprise CREMPs, collagen X, lysyl oxidase-like protein 2 and lysozyme; and more preferably CREMPs and collagen X represent at least 40% by weight of the total weight of the particles.

The ESM particles of the invention may be obtained by a process comprising a separation step (first step) and a size reduction step (second step).

The ESM particles of the invention may be separated (first step, or separation step) from other egg components by any convenient means. The eggs from which the ESM may be separated may be fertilised or unfertilised. The eggs may be intact, i.e. prior to hatching, or may be empty, i.e. the remnants of the egg following hatching or following extraction of the egg contents (albumen and yolk). Suitable means are for example described in WO2004/080428 and US 8580315. Preferably the ESM is prepared by the method for harvesting eggshell membrane in-line in commercial egg processing plants disclosed in WO2015/058790. In brief WO2015/058790 provides a method of processing eggshell residues, which emanate from an egg breaking unit and comprise eggshell portions as well as membrane portions, comprising feeding eggshell residues (e.g. having a particle size of about 0.5 mm to about 40 mm and a wet basis moisture content of about 3% to about 40%) from the egg breaking unit into a cyclone driven by a process gas having a temperature of less than about 85°C (preferably of less than about 60°C) and having a speed exceeding about 60 m/s (preferably between about 70 m/s and about 340 m/s). Within said cyclone vortex processing of the eggshell residues reduces particle size and peels said membrane portions off of said eggshell portions, such that said eggshell portions become separated from said membrane portions. Through a top outlet of said cyclone there is released mainly a mix of process gas, vapour and droplets, and through a bottom outlet of said cyclone there is released mainly a mixture of separated eggshell portions and membrane portions. Said released mixture is then separated into an eggshell portion part and a membrane portion part in a sorting device. The resultant ESM portion may then be processed further into the ESM particles of the invention as described herein, preferably with no intervening steps. In certain embodiments the method of preparing ESM comprises the further step of controlling time between feeding eggshell residues into and releasing said mixture out of said cyclone by adjusting an eggshell residue feed rate in relation to a total process gas feed rate, e.g. into an interval of about 0.5s to about 20s and preferably of about 1s to about 5s. In certain embodiments the method further comprises a step of centrifuging the eggshell residues prior to feeding them into said cyclone. In certain embodiments the feeding step is continuous. In other embodiments the sorting step comprises pneumatically expelling the membrane portion part off of sorting screens and out of the sorting device. The method may also comprise a final step of drying the membrane portion part.

ESM material in the form of flakes within the size range of around 1mm² to about 10mm² cannot be re-formed or processed into a sheet with the same structural characteristics as intact ESM. Preferably, the ESM particles are substantially that obtained from the shell-membrane separation process. In other words, the ESM particles of the invention are substantially chemically unmodified as compared to naturally occurring ESM from a corresponding avian source. More specifically the ESM particles are chemically substantially non-degraded, non-digested (e.g. chemically or enzymatically) and/or non-denatured as compared to naturally occurring ESM from a corresponding avian source. By "substantially non-degraded", it is meant that less than 20%, preferably less than 15%, 10%, 5% or 1% of the ESM components, show evidence of degradation as compared to naturally occurring ESM from a corresponding avian source. Non-digested and non-denatured should be interpreted accordingly. The degree of degradation/digestion/denaturation of ESM can be assessed by measuring the relative solubility of the ESM and/or the relative size or structure of the collagen fibres in the ESM. This may be achieved through routine techniques including immunohistochemistry/immunocytochemistry techniques and/or biomolecule (e.g. protein) stains and dyes.

In particular the ESM particles have not been exposed to a hydrolysis reaction or a disulphide bond reducing reaction, for example chemical or enzymatic, in particular an alkaline hydrolysis reaction. In other words the ESM particles of the invention are substantially non-hydrolysed, by which it is meant that less than 20%, preferably less than 15%, 10%, 5% or 1% of the ESM components show evidence of hydrolysis as compared to naturally occurring ESM from a corresponding avian source. The degree of hydrolysis of ESM can be assessed by measuring the relative solubility of the ESM and/or the relative size of the collagen fibres and/or the extent of collagen cross-linking in the ESM. This may be achieved through routine techniques including immunohistochemistry/immunocytochemistry techniques and/or protein stains and dyes.

Preferably the ESM particles of the invention are substantially, i.e. essentially, insoluble in water at a neutral pH, e.g. pH 6.8-7.2. For the purposes of the invention an insoluble material requires greater than 10L of solvent to dissolve 1g of solute.

After the separation step, the ESM particles of the invention may be obtained by a size reduction step (second step) : they may be prepared from ESM by any convenient particle size reduction, micronizing, grinding, pulverizing or milling technology means, e.g. ball milling, bead milling, jet milling, vortex milling, blade milling, rotor-stator dispersement, preferably followed by size selection, e.g. sieving and screening. The chosen particle size reduction method may be either performed dry or with a liquid medium which may or may not comprise other components of the scaffold. Cryo-pulverization may also be employed. In certain embodiments the particle size reduction process, and in certain embodiments the preceding ESM preparation process, is selected on the basis that ESM fibres of the required size (e.g. as recited above) are produced. Inter alia, pulverisation of dry ESM in a blade-mill and rotor-stator dispersement of a suspension of ESM flakes have been shown to be effective in this regard.

Thus, in accordance with the invention, a method for the preparation of the ESM particles may comprise providing ESM, and subjecting the ESM to a micronization process. Preferably the ESM is provided essentially free of non-ESM egg components and more preferably providing ESM essentially free of non-ESM egg components comprises separating ESM from non-ESM egg components as described in WO2015/058790 and washing the ESM so obtained with a weak acid solution (which term includes a weakly acidic solution), e.g. an aqueous solution of about 0.1% hydrochloric acid or acetic acid, thereby removing any residual calcium carbonate in the ESM. In other embodiments the micronized ESM is washed with said weak acid solution. This weak acid wash, especially treatment with an about 0.1% HCI solution, not only demineralises the ESM, thus minimising the amount of inorganic salts in the ESM, but also removes and/or inactivates infective agents, e.g. microorganisms (e.g. as described herein), prions and viruses.

### Additive(s)

The composition of the invention (i.e. hydrogel) may comprise at least one additive chosen from growth factors, differentiation factors, coloring agents, pharmacologic agents, biological markers and their mixtures.

Growth factors may be chosen from the transforming growth factors (such as TGF-beta 1, TGF-beta 2 or TGF-beta 3), epidermal growth factor (EGF), platelet derived growth factor-AB (PDGF-AB), Insulin-like Growth Factor-1 (IGF-1), vascular endothelial growth factor (VEGF) and fibroblast growth factor 2 (FGF-2, Fibroblast Growth Factor 2, also called basic Fibroblast Growth Factor or bFGF).

Growth/differentiation factors (GDF-1 to GDF-15) are members of the BMP family of TGF-beta superfamily proteins.

Preferably, the differentiation factor is chosen from GDF1, GDF2, GDF3, GDF4, GDF5, GDF6, GDF7, GDF8, GDF9, GDF10, GDF11, GDF12, GDF13, GDF14 and GDF15.

Coloring agents may be chosen from natural coloring agents and synthetic coloring agents. Natural coloring agents are produced from natural sources either from vegetable or mineral sources. Natural coloring agents may be chosen from mineral-derived coloring agents, such as Red Ferric Oxide, Titanium Oxide, Lead Oxide, Copper Sulfate or Carbon Black ; plant-derived coloring agents, such as Indigo, Beta-Carotene, Paprika, Turmeric, Curcumin, Caramel or Saffron ; and animal-derived coloring agents such as cochineal extract (and its lake, carmine), which is derived from an insect.

Synthetic coloring agents are produced by chemical synthesizing. Synthetic coloring agents may be chosen from Green S, Patent Blue V, Quinoline yellow, Carmoisine and Ponceau 4R.

Pharmacologic agents may be chosen from any active pharmaceutical ingredients, which may be used for a given pharmacologic effect. For example, the pharmacologic agent may be chosen from heparin, anti-inflammatory agents, beta-blockers. The pharmacologic agent will be chosen depending on the targeted purpose.

Biological markers may be chosen from immunohistochemistry markers such as bromodeoxyuridine (BrdU), cytokeratins, CD15, CD30, alpha feroprotein, CD117, CD10 or PSA (prostate specific antigen); and fluorescent markers such as fluorescein or rhodamine.

### Cells

The composition of the invention (i.e. hydrogel) may comprise isolated cells.

Said isolated cells may be any kind of cells. Preferably, they may be chosen from keratinocytes, fibroblasts, adipocytes and preadipocytes, endothelial cells, nerve cells, cells dermal dendritic cells, Langerhans cells, melanocytes, Merkel cells, sebocytes, macrophages, mast cells, epithelial cells of the hair follicles, the fibroblasts of the papilla of the hair follicle and induced pluripotent stem cells (iPSc).

They can also be chosen from lymphocytes (in particular B lymphocyte, T lymphocyte, cytotoxic T lymphocyte, NKT lymphocyte, regulatory T lymphocyte, helper lymphocyte), myeloid cells, granulocytes, basophilic granulocytes, eosinophilic granulocytes, granulocytes neutrophil, hypersegmented neutrophils, monocytes, macrophages, reticulocytes, trombocyte, mast cells, thrombocytes, megakaryocytes, dendritic cells, thyroid cells, thyroid epithelial cells, parafollicular cells, parathyroid cells, chief cells of the parathyroid gland, oxyphil cells, adrenal cells, chromaffin cells, pineal cells, glial cells, glioblasts, astrocytes, oligodendrocytes, microglia cells, neurosecretory magnocellular cells, stellate cells, Boettcher cells; pituitary cells, gonadotrophs, corticotrophs, thyrotrophs, somatotrophs, lactotrophs, lung cells (type I pneumocytes, type II pneumocytes, Clara cells); goblet cells, alveolar macrophages, myocardiocytes, pericytes, gastric chief cells, parietal cells, goblet cells, paneth cells, G cells, D cells, ECL cells, I cells, K cells, S cells, enteroendocrine cells, enterochromaffin cells, APUD cells), liver cells (hepatocytes, Kupffer cells), bone cells (osteoblasts, osteocytes, osteoclasts, odontoblasts, cementoblasts, ameloblasts), cartilage cells (chondroblasts, chondrocytes), hair cells (trichocytes), skin cells (keratinocytes, adipocytes, fibroblasts, melanocytes, nevus cells), muscle cells (myocytes, myoblasts, myotubes), tendon cells, kidney cells (podocytes, juxtaglomerular cells, mesangial intraglomerular cells, mesangial extraglomerular cells, cells of the macula densa), spermatozoa, sertoli cells, Leydig cells and oocytes.

The composition according to the invention may be used as an *in vitro* research model, or for obtaining a 2D (single layer) or 3D product, i.e. organoid or organ or tissue.

The hydrogel of the invention preferably comprises at least two hydrophilic thickening polymers, i.e. a first hydrophilic thickening polymer and a second hydrophilic thickening polymer. Preferably said first hydrophilic thickening polymer is alginate. Preferably said second hydrophilic thickening polymer is gelatin. Said hydrogel may be obtained by the following process :
A first step of mixing the first hydrophilic thickening polymer with an aqueous medium, preferably water, optionally by heating said mixture at a temperature comprised between 35°C and 45°C, until obtaining a homogenous first mixture;
A second step of mixing the second hydrophilic thickening polymer with an aqueous medium, preferably water, optionally by heating said mixture at a temperature comprised between 35°C and 45°C, until obtaining a homogenous second mixture;
Introducing ESM particles into the second mixture, in order to obtain an ESM particle second mixture ;
Mixing the ESM particle second mixture with the first mixture, until obtaining a homogenous hydrogel; and
Optionally introducing growth factors and/or differentiation factors and/or coloring agents and/or pharmacologic agents and/or biological markers and/or cells into the hydrogel. An object of the invention is also a hydrogel which is obtainable by the process described above.

The invention also relates to a bioink comprising a hydrogel of the invention, and isolated cells.

### Kit

The invention also relates to a kit comprising:
- a first composition, wherein said first composition is according to the invention (i.e. hydrogel or bioink as described); and
- a second composition which comprises, in a compatible medium, at least one crosslinking agent; and
- optionally a third composition comprising at least one compound chosen from cells, coloring agents, pharmacologic agents, differentiation factors, growth factors, biological markers and their mixtures.

The kit of the invention may be used as an *in vitro* research model, or for obtaining a 2D (single layer) or 3D product, i.e. organoid or organ or tissue.

The first composition of the kit is a composition or a bioink according to the invention. The second composition comprises, in a compatible medium, at least one crosslinking agent.

The third composition is optional, and comprises at least one compound chosen from cells, coloring agents, pharmacologic agents, differentiation factors, growth factors, biological markers and their mixtures. Said cells, coloring agents, pharmacologic agents, differentiation factors, growth factors, biological markers and/or their mixtures, may be as described above.

Each of the first, second and/or third composition is preferably formulated in a syringe.

The second composition comprises at least one crosslinking agent. Crosslinking may be physical, notably by ionic bonding, hydrogen bonding and/or hydrophobic effect, or chemical, notably by the formation of at least one covalent bond.

Physical crosslinking may be performed using polyvalent metal salts.

Chemical crosslinking may be performed using chemical crosslinking agents.

Preferably, the crosslinking agent is chosen from :
- polyvalent metal salts, preferably chosen from calcium, iron, silver, strontium, aluminum, manganese, selenium, copper and zinc, and
- chemical crosslinking agents, preferably chosen from carbodiimides, particularly water-soluble carbodiimides such as 1-ethyl-3-(3-dimethylaminopropyl)carbodiimide (EDC), N-cyclohexyl-N'-(2-morpholinoethyl)carbodiimide (CMC), dicyclohexylcarbodiimide (DCC), N-hydroxysuccinimide (NHS) and CDI (carbodiimidazole). The chemical crosslinking agent may also be chosen from photoreactive crosslinking polymers.

The polyvalent metal salt is preferably formulated into an aqueous solution. Preferably it is a solution of calcium chloride, gluconate or lactate ; or a solution of copper or zinc acetate, sulfate, chloride or gluconate.

Preferably, though not necessarily, the crosslinking is facilitated by an activator compound which reacts with the carboxylic acid group of the hydrophilic thickening polymer, preferably the alginate unit, to make it more reactive to the crosslinking agent. Useful activators for making a carboxylic acid group more reactive to the crosslinking agent, particularly an amine functional group of the crosslinking agent, are known in the art. Examples thereof include, but are not limited to, carbodiimides, particularly water-soluble carbodiimides such as, for example, 1-ethyl-3-(3-dimethylaminopropyl)carbodiimide (EDC), N-cyclohexyl-N'-(2-morpholinoethyl)carbodiimide (CMC), dicyclohexylcarbodiimide (DCC), N-hydroxysuccinimide (NHS) and CDI (carbodiimidazole).

Also preferred, when using an activator compound, is the use of a stabilizer for stabilizing the resulting activated group. Again, useful stabilizers for the activator groups are known in the art. For carbodiimides, particularly EDC, a useful stabilizer is 1-hydroxybenzotriazole (HOBT) which stabilizes the activated group against hydrolysis. Other useful stabilizers include N-hydroxysuccinimide and N-hydroxysulfylsuccinimide (sulfo-NHS).

### Bioprinting method

The invention also relates to a method for manufacturing a 2D or 3D product, comprising the following steps :
(a) preparing a composition or bioink of the invention, preferably by heating said composition to a temperature comprised between 35°C and 40°C and centrifugating it;
(b) introducing the composition obtained at the end of step (a) into at least one first cartridge of a bioprinter, said bioprinter comprising at least one printbed, at least one printhead linked to said first cartridge and having a nozzle, preferably two printheads each having a nozzle, and optionally a UV curing system; and
(c) manufacturing the 2D or 3D product in the bioprinter.

The manufacturing step (c) may be performed by means known in the art. The parameters may be adjusted to the targeted purpose. Preferably, the manufacturing step (c) is performed at a printhead temperature comprised between 35°C and 40°C, and a printbed temperature comprised between 3°C and 12°C. Preferably, it is performed at a printing pressure comprised between 25 and 35 KPa, and/or a nozzle speed comprised between 5 and 15 mm/s, and/or a nozzle inner diameter comprised between 0.23 to 0.45 mm.

By "bioprinting or "3D bioprinting", it is meant the process of creating cell patterns in a confined space using 3D printing technologies, where cell function and viability are preserved within the printed construct. Generally, 3D bioprinting utilizes the layer-by-layer method to deposit materials known as Bioinks to create tissue-like structures that are later used in medical and tissue engineering fields. There are currently at least three types of 3D bioprinting technologies: laser printing technology, extrusion technology and inkjet technology. In addition to these technologies, acoustic printer and hybrid printing technologies are emerging.

Preferably the 2D or 3D product is an organ, a tissue or an organoid.

Said method comprises a first step, i.e. step (a), which comprises the preparation of a composition or bioink of the invention to the following steps, and especially to its printing. Preferably step (a) comprises heating said composition to a temperature comprised between 35°C and 40°C. Preferably, it further comprises a centrifugation step. This allows eliminating air bubbles, and homogenizes the composition.

Step (a) preferably comprises preparing a composition or bioink of the invention by mixing, in an aqueous medium, at least one hydrophilic thickening polymer, preferably at least two hydrophilic thickening polymers, and the required particles of eggshell membrane, in an amount of less than 5% by weight of the total weight of the composition.

Preferably, the composition or bioink of the invention comprises a first and a second hydrophilic thickening polymers, and step (a) comprises:
i) dissolving the first hydrophilic thickening polymer in an aqueous medium, preferably water;
ii) introducing the required particles of eggshell membrane directly into the mixture of step i), or after mixing said particles in an aqueous medium (preferably water);
iii) dissolving the second hydrophilic thickening polymer in an aqueous medium, preferably water;
iv) introducing the mixture of step ii) into the mixture of step iii) to obtain a composition; and
v) optionally heating said composition of step iv) to a temperature comprised between 35°C and 40°C, and preferably further submitting it to a centrifugation step.
Preferably, the first hydrophilic thickening polymer is chosen from alginate and gelatin. Preferably, the second hydrophilic thickening polymer is different from the first hydrophilic thickening polymer and is chosen from alginate and gelatin.

Preferably, the first hydrophilic thickening polymer is gelatin, and the second hydrophilic thickening polymer is alginate.

Then, the composition obtained at the end of step (a) is introduced into at least one first cartridge of a bioprinter.

The bioprinter comprises at least one printbed, and at least one printhead linked to said first cartridge and having a nozzle. Preferably, the bioprinter comprises two printheads each having a nozzle. Preferably, the bioprinter comprises a UV curing system.

The bioprinter may be an inkjet, a laser or an extrusion-based bioprinter. Preferably the bioprinter is an extrusion-based bioprinter (i.e. an extruder).

The bioprinter is for example the Bio X of Cellink, which is a pneumatic driven system. The bioprinter may also be the Bio Assembly Bot 400 of Advanced Solutions.

Then, after step (b), the composition is extruded in the bioprinter and the 2D or 3D product is thus manufactured: it is step (c).

Step (c) is typically performed under conditions allowing a correct manufacture of the 2D or 3D product. Typically, step (c) is performed under the above listed conditions, i.e. at a printhead temperature comprised between 35°C and 40°C, a printbed temperature comprised between 3°C and 12°C, a printing pressure comprised between 25 and 35 KPa, a nozzle speed comprised between 5 and 15 mm/s, and a nozzle inner diameter comprised between 0.23 to 0.45 mm.

At the end of step (c), the desired 2D or 3D product is obtained.

Preferably, the 2D or 3D product obtained at the end of step (c) is mixed with at least one crosslinking agent. Said crosslinking agent is typically as described above.

When the crosslinking agent is chosen from polyvalent metal salts, then typically an aqueous solution of said polyvalent metal salt is poured onto the 2D or 3D product.

When the crosslinking agent is chosen from chemical photoreactive crosslinking agents, then preferably the bioprinter comprises a UV curing system. Said UV curing system comprises a UV lamp. The 2D or 3D product obtained at the end of step (c) is mixed with at least one chemical crosslinking agent, and put under UV light of the UV curing system.

Preferably, the method of the invention comprises a step of adding cells to the 2D or 3D product. Said addition of cells may be performed before or after the 2D or 3D product obtained at the end of step (c) is mixed with at least one crosslinking agent. When said addition of cells is performed before the 2D or 3D product obtained at the end of step (c) is mixed with at least one crosslinking agent, then the 2D or 3D product obtained after adding the crosslinking agent is a fixed structure already comprising cells and hydrogel. When said addition of cells is performed after the 2D or 3D product obtained at the end of step (c) is mixed with at least one crosslinking agent, then the 2D or 3D product obtained after adding the crosslinking agent and the cells is a fixed hydrogel structure enriched with cells.

Preferably:
- cells are added during step (a) or the composition already comprises cells, or
- cells are introduced into at least one second cartridge of a bioprinter, said bioprinter comprising two printheads, a first printhead being linked to the first cartridge and a second printhead being linked to said second cartridge, and cells are bioprinted during step (c) or after step (c), or
- cells are added after step (c) once the 2D or 3D product is obtained. For example, according to this embodiment, cells may be added manually to the 2D or 3D product, or by using a specific equipment, such as an arm-robot comprising a syringe containing said cells.

The invention also relates to an isolated 2D (single layer) or 3D organ or tissue which is obtainable by a method as described above.

The invention is now illustrated by the following examples.

### Examples

In the following examples, the « PEP » are the eggshell membrane particles or ESM particles.

For all studies the inventors used CAD models described in table 1.

**Table 1. Structures designed and printed results**

| Model | Designed structures | W*D*H (mm) | Layers | Layer height (mm) |
|---|---|---|---|---|
| Model A | 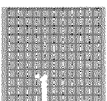 | 30*30*1.23 | 1 | 1.23 |
| Model B | 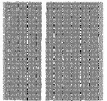 | 30*30*1.23 | 3 | 0.41 |
| | | | | 0.41 |
| | | | | 0.41 |

### Example 1 : Formulation, Printability and primary degradation test for hydrogel Alginate 2.5%-Gelatin 3%-Cellulose 2%-0.5% PEP (invention)

### I- Formulation method

For Alginate 2.5%-Gelatin 3%-Cellulose 2%-0.5% PEP 50 ml (invention): 1.25g ASH was dissolved in 35 ml H₂O for 2 h at 37 °C until it was completely dissolved. 1500 mg of gelatin (Porcine, type A, 300 blooms) was added in 10 ml of deionized H₂O at 37°C and stirred for 30 min until it was completely dissolved. 250 mg of PEP was added into gelatin solution and stirred for 1h at 37 °C. The gelatin+PEP solution was added into the alginate solution under stirring. 5 ml of H₂O deionized was used for rinse. The mixture was stirred for additional 1h at 37°C. 1 g of hydroxypropyl cellulose was added in small portion to alginate-gelatin-PEP mixture and stirred for 2h at 37°C.

### II- Printing method

The hydrogel was heated for 1h in water bath 37°C. 5ml hydrogel was loaded into a syringe and then introduced to 3 ml plastic cartridge of Cellink Bio X. After being centrifuged at 4900 rpm for 10 min until air bubbles were fully eliminated, the cartridge was installed into the printhead of Cellink Bio X which was set to 37°C. Then a non-sterile high precision blunt conical nozzle 22G was fixed to the cartridge. The printbed was cooled to 10°C to preserve the shape of the scaffold fabricated. The printing was started only in 30 min to ensure the temperature equilibration between the cartridge and the printhead.

Two models were used: the "model A" and the "model B", designed in previous work, were subjected to printing test and the 1^{st} layer height was set to 100%. The hydrogel flow was tested under different air pressure from 15-30 KPa. The printability assay was carried out with the minimum pressure which can generate constant and continuous extrusion. 2 feed rates, 5mm/s and 10 mm/s, were adapted.

After printing, to avoid the deformation, the dimension, the strand width and the pore size of the scaffold fabricated were measured immediately and as quickly as possible by Keyence IM-7030T at multiple locations (at least 10) and averaged. Then the whole structure was immerged into 2% CaCl₂ solution for 15 min, washed 3 times by deionized H₂O. The crosslinked structure was measured again in the same manner by Keyence IM-7030T.

### III- Results for Alginate 2.5%-Gelatin 3%-Cellulose 2%-0.5% PEP (invention)

For hydrogel Alginate 2.5%-Gelatin 3%-Cellulose 2%-0.5% PEP, its printability was tested with 2 models: model A and model B. Both models were successfully printed under 30KPa at 5 mm/s with good quality.

The model A was created with dimension 30.36 mm, strand width 0.861 mm and pore size 1.829 mm respectively. After crosslinking in CaCl₂, the dimension, the strand width and the pore size were 28.97 mm, 0.710 mm and 1.848 mm respectively.

The model B was printed with a dimension 30.68 mm, a strand width 1.012 and 0.900 mm and a pore size 0.442 mm, respectively. After crosslinking they were 27.05 mm, 0.964 mm, 0.857 mm and 0.410 mm respectively.

### IV- Primary degradation test in PBS at r.t. (room temperature)

### Test method:

After printing, the scaffolds (experimental dimension about 27*27*0 .8mm, strand width about 1mm, pore size about 0.6 mm) were washed with deionized H₂O 5 times. Then they were immerged in 5 ml PBS at r.t in a 6-well plate.

### Results and conclusion:

After 1-day incubation in PBS, the structure integrity of all scaffolds were almost preserved, but they could not be manipulated with a tweezer anymore. From the 3^{rd} day, the scaffolds began to break obviously into small pieces. The structures of model A seemed to have a lower dissolving rate in PBS, thus more resistant compared to the structures of model B. At day 7, all the structures were considered completely dissolved in PBS.

### Conclusion:

In this study, the printability of hydrogel Alginate 2.5%-Gelatin 3%-Cellulose 2%-0.5% PEP was investigated using the model B and the model A. Results obtained showed its potential as suitable bioink in 3D printing technology applications. The printed scaffolds displayed similar printing quality and degradation behaviour as other hydrogels prepared in the work.

### Example 2:

### I- Models designed through CAD software

In total, 8 Models were designed (Table 1). They have the same depth and width 30x30mm, but different height either 1 mm or 1.23 mm. Furthermore, they have the same strand width 0.41 mm which correspond to the inner diameter of the nozzle 22G used in the subsequent printing process. The inventors can roughly classify these 8 models into 2 groups according to the model height:
- Group 1, including the model 7, 14, 6, and 8, with a model height 1mm;
- Group 2, including the model A, B, 10 and 11, with a model height 1.23mm.

**Table 2**

| Model | W*D*H (mm) | Layers | Layer height (mm) | Middle layer | Strand width (mm) | Pore size (mm) | During printing | |
|---|---|---|---|---|---|---|---|---|
| | | | | | | | 1^{st} layer setting | Number of layers sliced |
| Model 7 | 30*30*1 | 1 | 1 | / | 0.41 | 2.28 | 66% | 3 |
| Model 14 | 30*30*1 Fillets 0.5 | 1 | 1 | / | 0.41 | 2.28 | 66% | 3 |
| Model 6 | 30*30*1 | 3 | 0.33 | Displaced | 0.41 | 2.28 | 66% | 3 |
| | | | 0.33 | | | 0.935 | | |
| | | | 0.34 | | | | | |
| Model 8 | 30*30*1 | 3 | 0.27 | Displaced | 0.41 | 2.28 | 66% | 3 |
| | | | 0.365 | | | | | |
| | | | 0.365 | | | 0.935 | | |
| Model A | 30*30*1.23 | 1 | 1.23 | / | 0.41 | 2.28 | 100% | 3 |
| Model B | 30*30*1.23 | 3 | 0.41 | Displaced | 0.41 | 2.28 | 100% | 3 |
| | | | 0.41 | | | | | |
| | | | 0.41 | | | 0.935 | | |
| Model 10 | 30*30*1.23 | 3 | 0.41 | Displaced and smaller | 0.41 | 2.28 | 100% | 3 |
| | | | 0.41 | | | 0.935 | | |
| | | | 0.41 | | | | | |
| Model 11 | 30*30*1.23 | 3 | 0.41 | rotated 90° | 0.41 | 2.28 | 100% | 3 |
| | | | 0.41 | | | | | |
| | | | 0.41 | | | | | |

In group 1, model 7 is the simplest, with dimension 30×30×1mm, strand width 0.41mm, pore size 2.28 mm, and without stratification. During the printing process, the 1^{st} layer height is set to 66%, i.e 0.41 x66%=0.27 mm. The house software of 3D printer Cellink Bio X used in this work will automatically slice the model into 3 layers. It seems that the printing quality of the sharp angles can be improved if they are filleted in x-y plane in the step of the design. Therefore, model 14 was designed in the presence of rounded corners sized to 0.5 mm everywhere.

Models 6 and 8 were designed with 3 layers and a displaced middle layer. This kind of model has two types of pore size from top view: 0.935 mm created by 2 displaced layers and 2.28 mm within each layer. The model 6 and the model 8 are characterised from the layer height. The model 6 has three identical layer height, 0.33, 0.33 and 0.34 mm. For the model 8, the 1^{st} layer height is 0.27mm. The other two layers share a same layer height 0.365 mm.

In group 2, all models have a model height 1.23 mm. The model A has no stratification with a pore size 0.28 mm, forming an analogous with the model 7 in the group 1. They are distinct at the model height. The other 3 models in the group 2, i.e. models B, 10 and 11, contain 3 layers and each layer is in 0.41mm height. The model B was designed in order to compare with the model 6 and the model 8 in the group 1. The model 10 of which the intermediate layer is smaller probably will display a different edge printing quality compared to the model 10. The model 11 has a quarter-turned (90°) intermediate layer, creating various pore shape and size from top view. For these 4 models, during printing, the 1^{st} layer height is set to 100%, i.e. 0.41 mm. The models are sliced automatically into 3 layers.

### II- Hydrogel preparation

### Prepare the hydrogel 2.5%ASH+3%Gelatin 100ml (comparative)

The Alg-Gel hydrogel was prepared as the following steps. Firstly, 2.5g ASH powder was dissolved into 70 ml deionized H₂O and stirred with an electric stirrer at 37 °C, until it was completely dissolved. Secondly, 3.0g gelatin particles were dissolved into 20 ml deionized H₂O at 40 °C water bath for 30 min which allowed gelatin to completely liquefy. Thirdly, the gelatin solution was added into the ASH solution under stirring and the spare 10 ml deionized H₂O was used to rinse. The mixture was stirred for 2h at 37 °C in a water bath. The gel was then kept in the fridge.

### Prepare the hydrogel 2.5%ASH+3%Gelatin+0.25/0.5/1.0/1.5% PEP 50ml (invention)

### Comparison between two preparation protocols

### Method 1: 2.5%ASH+1%PEP+3%Gelatin

Firstly, 1.25g ASH powder was dissolved into 35 ml deionized H₂O under electric stirrer for 3h at 37 °C, until it was completely dissolved. 0.5g PEP in 5ml deionized H₂O was stirred with magnetic stirrer during 1h until no visible PEP particles were observed. Then the PEP solution was added into ASH gel and the mixture was stirred for 3h at 37 °C. 1.5g Gelatin in 5ml deionized H₂O was stirred at 40 °C for 1 h which allowed gelatin solution to completely liquefy. Gelation gel was introduced into the ASH+PEP mixture. The spare 5ml deionized H₂O was used to rinse. The ASH+PEP+Gelatin mixture was maintained under stirring for 2h at 37 °C. Since many PEP particles were still observed in the mixture, the mixture was stirred for an additional 3h at 37 °C, but no improvement was gained at the end.

### Method 2: 2.5%ASH+(3% Gelatin+1% PEP) 50ml

1.25g ASH powder was dissolved into 30 ml deionized H₂O under electric stirrer for 3h at 37 °C, until it was completely dissolved. 1.5g Gelatin in 10 ml deionized H₂O was stirred for 30 min at 40 °C which allowed gelatin solution to completely liquefy. 0.5g PEP was added into gelatin solution and was stirred with magnetic stirrer during 2h until no visible PEP particles observed. Then the Gelatin + PEP mixture was added into ASH gel. The rest 10ml deionized H₂O was used to rinse. The new mixture was stirred for 3h at 37 °C. Through this preparation method, no huge PEP particles were observed in the mixture.

In summary, compared to method 1, via method 2 the PEP was better dispersed in the hydrogel. As the existence of PEP particles in hydrogel can result in nozzle jamming during the printing process, method 2 was selected to prepare all the 4 blends of ASH+Gelatin+PEP mixtures needed in the following work:
2.5%ASH+3%Gelatin+0.25% PEP,
2.5%ASH+3%Gelatin+0.5% PEP,
2.5%ASH+3%Gelatin+1.0% PEP and
2.5%ASH+3%Gelatin+1.5% PEP.

### III- 3D Printing method

To investigate the printability of the model designed and the hydrogel prepared above, the inventors examined the dimensions, the pore size, and the strand diameter of the scaffolds printed by Cellink Bio X (pneumatic driven system) using Keyence IM-7030T. The 8 models designed above were subjected to printing test firstly using the hydrogel 2.5% ASH +3% Gelatin under following printing parameters: standard nozzle 22G, printhead 37°C, printbed 10°C-12°C, pressure 25/30/35/40KPa, and feed rate 5/10mm/s. The standard nozzle 22G (inter diameter 0.41mm) was used because it is the most common nozzle size used and it provides a good balance between print speed and precision. The printhead was heated to 37°C, according with the melting point of gelatin. The last but the most important, the printbed should be cooled to at least 10 °C to preserve the shape of the scaffold fabricated.

Related to experiments, the hydrogel was heated for 1h in 37 °C water bath. 5ml hydrogel were loaded into a syringe and then introduced to 3 ml plastic cartridge of Bio X. After being centrifuged at 5000 rpm for 10 min to reduce air bubbles, the cartridge was installed into the printhead of Cellink Bio X which was set to 37 °C. After 30 min, the cartridge was centrifuged again for 10 min at 5000 rpm if necessary. Then the cartridge was put into the printhead and a non-sterile high precision blunt nozzle 22G was fixed to the cartridge. The printing was started only in 30 min to ensure the temperature equilibration between the cartridge and the printhead.

During printing, the 1^{st} layer height was set either 66% or 100% depending on the chosen model. The printbed was cooled to around 10 °C. The hydrogel flow was tested under different air pressure from 25, 30, 35 to 40 KPa. The printability assay was started with the minimum pressure which can generate constant and continuous extrusion. 2 feed rates, 5mm/s and 10 mm/s, were tested.

After printing, the dimension, to avoid the deformation, the strand width and the pore size of the scaffold fabricated were measured immediately and as quickly as possible by Keyence IM-7030T at multiple locations (at least 10) and averaged. Then the whole structure was immerged into 2% CaCl₂ solution for 15 mins, washed 3 times by deionized H₂O. The crosslinked structure was measured in the same manner by Keyence IM-7030T.

### IV- Model Printability evaluation using hydrogel 2.5% ASH + 3% Gelatin

### For models of group 1 30*30*1mm

The model 7 was printed with air pressure 35KPa and feed rate 5mm/s. The dimension of the scaffold printed were 30.84*31.22 mm, which are comparable to the designed values 30*30 mm. However, as in previous work, the strand width 1.294 mm and the pore size 1.370 mm were found significantly different to theoretical values 0.41mm and 2.28mm respectively. After crosslinking, the dimensions were reduced to 26.72*26.90 mm. The strand width and the pore size became 1.103 mm and 1.246 mm.

For the model 14 with filleted corners, when the 1^{st} layer height was set to 66%, the model was sliced into 3 layers. The 1^{st} layer looked normal, but the 2nd and the 3^{rd} layer were sliced unusually. Thus, only the 1^{st} layer was successfully printed. From the 2^{nd} layer, the printing proceeded as it was sliced, resulting in printing failure. When the 1^{st} layer height setting was changed to 100%, the model was badly sliced into 2 layers.

The model 6 was printed with air pressure 35 KPa and feed rate 5 mm/s. The dimension of the scaffold printed were 30.40*30.72 mm. Distinguished to model 7, the model 6 had 2 kinds of experimental beam width, the larger one due to the overlapping of 2 layers (the first and the third layer) and the thinner one corresponding to the strand width of intermediate single layer. As mentioned previously, from top view, model 6 have two kinds of pore as well. The strand width and pore size are measurable only if there are pores through. As a result, for some scaffold printed, when the strand width was too large that the pores are not through anymore, we could not measure the pore size. That is the reason why in the printing test of model 6, the inventors only obtained the single layer strand width 0.633 mm and the pore size 0.409 mm. After crosslinking, the dimension, the strand width and the pore size were 27.10*26.91 mm, 0.600 mm and 0.381 mm respectively.

The model 8 was successfully fabricated with air pressure 25 KPa and 30 KPa. The feed rate was set to 5 mm/s. Two scaffolds obtained with different air pressure but a same feed rate 5 mm/s showed comparable dimension (30.34*30.59 mm vs 30.54*30.27 mm), strand width (1.211 and 0.661 mm vs 1.168 and 0.579 mm) and pore size (0.527 mm vs 0.602 mm). After crosslinking, the structure maintained comparable. Increasing the feed rate from 5 mm/s to 10 mm/s, leading to failed printing or large strand width.

### For models of group 2 30*30*1.23mm

The model A was printed under 30 KPa and feed rate 5 mm/s. The scaffold has the dimension 30.38*30.56 mm, the strand width 0.931 mm and the pore size 1.808 mm. After crosslinking, the dimension was 26.22*26.40 mm, the strand width was 0.691 mm and pore size 1.641 mm. Increasing the pressure from 30 to 35 KPa, the scaffold was obtained with obviously greater dimension and strand width and resulted smaller pore size because of over extrusion. By contrast, doubling the feed rate led to printing imperfections even failures.

The model B could be well printed under 35 KPa with feed rate 5 mm/s or 10 mm/s. Surprisingly, with 2 different feed rates, the scaffolds displayed nearly similar dimension about 30.5*30.5 mm, strand width about 1.000 mm and 0.6 mm, and pore size about 0.5 mm. After crosslinking, the dimension, the strand width and the pore size were around 26.5*26.5mm, 0.90 mm and 0.55 mm, and 0.48 mm respectively.

The models 10 and 11 were unfortunately not printed as expected, because the layer did not adhere well to the layer, especially on the edge, no matter what air pressure and feed rate were adopted.

### Discussion and conclusion

Among the 8 models designed, 3 models including the model 14, the model 10 and the model 11 were not printable. The model 14 could not be sliced by the house software of the printer used. The model 10 and the model 11 had bad adhesion between layers because many parts lacked support. Modifications were needed to turn these models printable.

The other 5 models were all printable. In the group 1, comparing with the model 6, the model 8 seems more advantage. The former was printed only if the air pressure was increased to 35 KPa and the strand width of the scaffold printed was too large resulting in unobservable pores, whereas the later could be printed under a lower air pressure 25 KPa or 30 KPa with thinner strand width and clear pores.

As regarding the model 7 and the model A, the model A could be printed from a lower pressure 30 KPa. Under the same air pressure and feed rate (35 KPa and 5 mm/s), scaffolds printed for these two models displayed comparable dimension (30.8*31 mm), strand width (1.3 mm and 1.1 mm) and pore size (1.4 mm).

Though the model B was printed with air pressure 25 and 30 KPa and the model 8 was printed with a higher pressure 35 KPa, the dimension, the strand width and the pore size of the scaffolds printed were almost in the same order, without obvious differences.

In one word, based on this model printability evaluation, 4 models, the models 7 and 8 in group 1 and the models A and B in group 2, were selected to study the printability of the PEP-contained hydrogels.

### V- Printability of PEP-contained hydrogels

### For hydrogel 2.5% ASH +3% Gelatin +0.25% PEP

Using hydrogel 2.5% ASH +3% Gelatin +0.25% PEP, both models 7 and A were printed with air pressure 35 KPa and feed rate 5 mm/s. The scaffolds printed had nearly the same dimension (30.52*30.89 mm vs 30.72*31.01 mm), slightly different strand width (1.414 mm vs 1.209 mm). Due to the greater strand width, the scaffold of the model 7 had bigger pores size. For the model A, the printability was also tested under 40 KPa and 5 mm/s. Surprisingly, the extra extrusion of the hydrogel did not produce larger strand width inhere.

For the model 8, only under 35 KPa and 5 mm/s, the printing was reluctantly completed, but not measurable.

By contrast, the model B was easier to print, with either 30 KPa and 5 mm/s or 35 KPa and 5 mm/s. The scaffold obtained had close dimension in the range of 30.5*31.5 mm and pore size in the range of 0.55 mm. While the air pressure was maintained to 35 KPa, enhancing the feed rate from 5 mm/s to 10 mm/s resulted in the decreased strand width from 1.0 mm to 0.9 mm and resulted increased pore size from 0.5 mm to 0.6 mm.

### For hydrogel 2.5% ASH +3% Gelatin +0.5% PEP

The model 7 was printed under 30 KPa et 5 mm/s. The scaffold printed had dimension 31.26*31.73 mm.

The strand width and the pore size were around 1.351 and 1.633 mm. With this hydrogel, the printing was also possible under pressure 35 KPa and feed rate 10 mm/s. And it was not surprising that the dimension 30.60*30.73 mm and the strand width 1.173 mm were closer to the values designed, compared to the structure obtained under 30 KPa and 5 mm/s, because it is known that combining a fast extrusion and a high feed rate can generate the same even better printing results as long as the rheological properties of the hydrogel allow a successful printing.

The model A was printed under either 30 KPa and feed rate 5 mm/s or 35 KPa and feed rate 5 mm/s. The scaffold printed had closed dimension, strand width, as well as pore size: 30.82*30.58 mm vs 30.56*30.74, 1.039 mm vs 1.139 mm, 1.658 mm vs 1.597 mm.

The model 8 in the group 1 were printable under 30 or 35 KPa with feed rate 5 mm/s, however, caused by over extrusion the strand width and the pore size of the scaffolds printed were not able to be measured. Nevertheless, reducing the air pressure from 30 KPa to 25 KPa brought about failure printing.

The printing of its analogous model, the model B, was achieved with pretty pores under the same conditions 30/35 KPa. The dimension, the strand width and the pore size of scaffolds obtained were similar, in the range of 30.3*30.6 mm, 0.959-1.058 mm and 0.498-0.574 mm.

### For hydrogel 2.5% ASH +3% Gelatin +1.0% PEP

The model 7 was printed under 30KPa and feed rate 5 mm/s. The scaffold printed had dimension in the order of 31.60*31.26 mm. The strand width and the pore size were in the range of 1.121 and 1.480 mm. The printing was able to be carried out as well under pressure 35 KPa with feed rate 5 or 10 mm/s. However, both 2 scaffolds reached under 35 KPa presented obviously larger dimension and strand width.

In the case of model A, the printing could be accomplished only under 35 KPa and feed rate 5 mm/s, with dimension 32.02*31.78 mm, strand width 1.421 mm and pore size 1.474 mm.

The model 8 in the group 1 were not printable until the pressure was increased to 35 KPa. With feed rate 5 mm/s, the strand width and the pore size of the scaffold printed were not able to be measured. Increasing the feed rate from 5 to 10mm/s yielded an interesting scaffold with dimension of 30.39*30.69 mm, strand width of 1.061 mm and pore size of 0.564 mm.

The printing of its analogous model, the model B, was achieved with pretty pores under the conditions 35 KPa and feed rate 5 mm/s. The dimension, the strand width and the pore size of scaffolds obtained were 30.52*30.65 mm, 0.887 mm and 0.696 mm.

### For hydrogel 2.5% ASH +3% Gelatin +1.5% PEP

The model 7 was printed under 35 KPa and feed rate 5 mm/s. The scaffold printed had dimension 30.54*30.50 mm.

The strand width and the pore size were in the range of 1.108 and 1.614 mm. The printing was able to be carried out as well at 40 KPa with feed rate 5 or 10 mm/s. With feed rate 5 mm/s, the strand width and the pore size of the scaffold printed were not able to be measured. Increasing the feed rate from 5 to 10 mm/s yielded a scaffold with dimension of 30.92*30.80 mm, strand width 1.180 mm and pore size 1.429 mm.

The model A was printed under 35 KPa and feed rate 5 mm/s. The scaffold printed had dimension 30.21*30.55 mm.

The strand width and the pore size were in the range of 0.918 and 1.925 mm. The printing was able to be carried out as well at 40 KPa with feed rate 5 or 10 mm/s, yielding 2 scaffolds with dimension and strand width larger than those under 35 KPa.

The model 8 in the group 1 were not printable unless the pressure was increased to 35 KPa with feed rate 5mm/s, the scaffold printed displayed dimension of 30.67*30.59 mm, strand width 1.248 mm and pore size 0.613 mm. Increasing the pressure to 40 KPa, under feed rate 5 or 10 mm/s, the scaffolds obtained were not measurable.

The fabrication of the analogous model B was achieved with pretty pores from 35 KPa. The feed rate could be set to 5 mm/s or 10 mm/s. The dimension of the 4 scaffolds obtained ranged from 30.44 to 30.92 mm, the strand width ranged from 0.983 to 1.225 mm and the pore size ranged from 0.505 to 0.659 mm.

### Discussion and conclusion

The results in this part of work revealed that all the 5 hydrogels prepared were printable under appropriate air pressure and feed rate. Generally speaking, most of the successful printings were achieved with air pressure 30 or 35 KPa and feed rate 5 mm/s. The presence of PEP in the hydrogel did not influence obviously the hydrogel printability, but it seems that the texture of the PEP-contained hydrogel was visually less viscous and more rigid compared to the hydrogel without PEP (rheological studies were needed).

Among the 4 models used in this study, model B produced the most stable scaffolds with interesting pore size. Therefore, the printing parameters together with the measurement of the model B fabricated using 5 hydrogels prepared were collected and showed in table 3.

**Table 3**

| Hydrogel 2.5%ASH + 3%Gelatin | Printing parameters | | | Dimensio n (mm) | Strand width (mm) | | Pore size (mm) | |
|---|---|---|---|---|---|---|---|---|
| | Pressur e (KPa) | Feed rate (mm/s ) | 1^{st} layer heigh t (mm) | | 2 layer s | singl e layer | Before crosslinkin g | After crosslinkin g |
| without PEP | 35 | 5 | 100% | 30.66 | 1.074 | 0.683 | 0.498 | 0.459 |
| +0.25% PEP | 30 | 5 | 100% | 30.74 | 1.020 | 0.593 | 0.568 | 0.514 |
| +0.5% PEP | 30 | 5 | 100% | 30.48 | 1.058 | 0.665 | 0.498 | 0.563 |
| +1.0 % PEP | 35 | 5 | 100% | 30.58 | 0.887 | 0.624 | 0.696 | 0.637 |
| +1.5% PEP | 35 | 5 | 100% | 30.78 | 1.225 | 0.631 | 0.506 | 0.458 |

From this table, the inventors observed that the hydrogel containing a high concentration of PEP (1% and 1.5%) required a slightly higher air pressure (35 KPa). The concentration of PEP did not affect significantly the dimensions, the strand width or the pore size of the scaffolds fabricated. All the five scaffolds obtained displayed dimension around 30.5 mm *30.5 mm, very closed to the value designed 30*30 mm. The strand width of single layer ranged from 0.593 mm to 0.683 mm and the pore size before crosslinking ranged from 0.498 mm to 0.696 mm, respecting less the theoretical value 0.41 mm and 2.28 mm. However, the most important point in here is that after crosslinking the experimental pore size ranging 0.45-0.65 mm was in agreement with the optimal value reported in literature for cell culture in the field of 3D bio fabrication.

### Conclusion

The printability of models designed by CAD software and 5 alginate-gelatin hydrogels without or with PEP was investigated. The appropriate printing parameters were determined. Finally, some promising scaffolds with pore size around 500 µm were obtained.

### Example 3: Formulation, printability test and primary degradation test for hydrogel Chitosan 1.6 %-Gelatin 3.2% (comparative) and Chitosan 1.6%-Gelatin3.2%-PEP 0.5% (invention)

### I- Formulation method:

Chitosan-hydrogel without PEP: 600 mg of chitosan (100-300 kDa, ≥75 deacetylation) was dissolved in 5 ml acetic acid 1M in a closed container under magnetic stirring with low speed (to avoid air bubbles) for 1 h at r.t until chitosan was completely dissolved. Then 15 ml of deionized H₂O was added dropwise and stirred for additional 30 mins (pH measured = 4.4). NaOH 0.5M (~ 1.2 ml) was added dropwise into the solution of chitosan under stirring until pH = 4.7. 1200 mg of gelatin (Porcine, type A, 300 blooms) was added in 10 ml of deionized H₂O at 40°C and stirred for 30 min until it was completely dissolved. The gelatin solution was added dropwise into the chitosan solution under stirring (pH measured = 4.8). 5 ml of deionized H₂O was used for washing. NaOH 0.5M (~ 2ml) was added dropwise into the chitosan-gelatin mixture under stirring, until pH was around 6.0 without chitosan precipitation. The mixture was set by side for one night at r.t. Thus, V=38ml; Cchitosan weight/volume = 1.6%; Cgelatin weight/volume = 3.2%.

Chitosan-hydrogel with PEP: To prepare Chitosan-Gelatin-PEP hydrogel, a Chitosan-Gelatin mixture was prepared firstly as described above. 600 mg of chitosan (100-300 kDa, ≥75 deacetylation) was dissolved in 5 ml acetic acid 1M in a closed container under magnetic stirring with low speed (to avoid air bubbles) for 1 h at r.t until chitosan was completely dissolved. Then 15 ml of deionized H₂O was added dropwise and stirred for additional 30 mins (pH measured = 4.4). NaOH 0.5M (~ 1.2 ml) was added dropwise into the solution of chitosan under stirring until pH = 4.7. 1200 mg of gelatin (Porcine, type A, 300 blooms) was added in 10 ml of deionized H₂O at 40°C and stirred for 30 min until it was completely dissolved. The gelatin solution was added dropwise into the chitosan solution under stirring (pH measured = 4.8). NaOH 0.5M (~ 2ml) was added dropwise into the chitosan-gelatin mixture under stirring, until pH was around 6.0 without chitosan precipitation. The mixture was set by side for one night at r.t. 5 ml of deionized H₂O was used for washing the container of gelatin. 200 mg of PEP was added in this 5 ml of water and stirred for 2h. Then the solution of PEP was added into the chitosan-gelatin hydrogel prepared and well mixed. V=38ml; Cchitosan w/v =1.6%; Cgelatin w/v=3.2%; CPEP=0.5%.

### II- Printing method

The hydrogel was heated for 1h in water bath 30°C. 5ml hydrogel was loaded into a syringe and then introduced to 3 ml plastic cartridge of Cellink Bio X. After being centrifuged at 4900 rpm for 10 min to reduce air bubbles, the cartridge was installed into the printhead of Cellink Bio X which was set to 30 °C. After 30 min, the cartridge needed probably to be centrifuged again for 10 min at 4900 rpm. Then the cartridge was put into the printhead and a non-sterile high precision blunt needle 22G was fixed to the cartridge. The printbed was cooled to at least 10 °C to preserve the shape of the scaffold fabricated. The printing was started only in 30 min to ensure the temperature equilibration between the cartridge and the printhead. A cylindric model 10*10*3 mm was subjected to printing test with the following parameters:

| Gel | Needle | T Print Head (°C) | T Print Bead (°C) | Pressure (KPa) | 1^{st} layer height | Feed rate (mm/s) | Infill pattern | Infill Density | Layers |
|---|---|---|---|---|---|---|---|---|---|
| Chitosan-hydrogel without PEP | 22G | 30 | 10 | 70 | 66% | 5 | rectilinear | 50% | 5 |
| Chitosan-hydrogel with PEP | 22G | 30 | 10 | 30 | 66% | 5 | rectilinear | 50% | 5 |

### Post-printing crosslinking in TPP

After printing, to avoid the deformation, the scaffold fabricated were kept in refrigerator at 4°C for 10 min. Then they were immerged in 10 % TPP w/v at 4°C for 1h and washed 3 times with deionized H₂O.

### III- Primary degradation test in PBS of the scaffolds crosslinked in TPP

**Method:** After crosslinking, the structures were taken out by a pair of tweezers, washed by PBS for 3/5 times to remove residual TPP. Then they were immersed into 1ml of PBS and put in the water bath incubator at 37 °C.

**Results and conclusion:** After 8 days of incubation, the scaffolds printed with Chitosan 1.6%-Gelatin 3.2%-PEP 0.5% of the invention maintained their integrity and could be manipulated with a tweezer.

In contrast, the scaffolds printed with Chitosan 1.6%-Gelatin 3.2% (comparative) lose its shape. At day 9, the scaffolds printed with Chitosan 1.6%-Gelatin 3.2% were broken into small pieces.

The scaffolds printed with Chitosan 1.6%-Gelatin 3.2%-PEP 0.5% stayed complete for more than 20 days. According to this primary test, the presence of PEP slowed significantly the degradation rate of chitosan-gelatin hydrogel in PBS at 37°C.

### Example 4: Protocol for bioprinting

A typical protocol is as follows:
The desired model is subjected to printing test firstly using hydrogel via Cellink Bio X (pneumatic driven system) with following printing parameters: standard nozzle 22G, printhead 37°C, printbed 10°C-12°C, pressure 25/30/35/40KPa, feed rate 5/10mm/s.

The gel is heated for 1h in water bath 37 °C. 5ml gel is loaded into a syringe and then introduced to 3 ml plastic cartridge of Bio X. After being centrifuged at 5000 rpm for 10 min, the cartridge is installed into the printhead of Cellink Bio X which was set to 37 °C. After 30 mins, the cartridge is centrifuged again for 10 mins at 5000 rpm. Then the cartridge is put into the printhead and a non-sterile high precision blunt nozzle 22G is fixed to the cartridge. The printing is started only in 30 min to ensure the temperature equilibration between the cartridge and the printhead.

During printing, the 1^{st} layer height is set either 66% or 100% depending on the model chosen. The printbed is cooled to around 10°C. The hydrogel flow is tested under different air pressure from 25, 30, 35 to 40 KPa. The minimum pressure which can generate constant and continuous extrusion is selected. Two feed rate 5 mm/s and 10 mm/s are tested.

After printing, the dimension, the strand width and the pore size of the structure are measured immediately and as quickly as possible by Keyence IM-7030T at multiple locations (at least 10) and averaged. Then the whole structure is immerged into 2% CaCl₂ solution for 15 min, washed 3 times by deionized H₂O. The crosslinked structure is measured again by Keyence IM-7030T.

### Example 5 : Other 3D structures

Besides the models as described above, it was also possible to bioprint the following models, with the disclosed equipment (photos are not shown):

| **Equipment** | **Structure details** |
|---|---|
| BioAssemblyBot Advanced 400 from Advanced Solutions Life Sciences (extrusion method) | Half-sphere structure with a diameter of 5 mm (at the base) |
| BioAssemblyBot Advanced 400 from Advanced Solutions Life Sciences (extrusion method) BioAssemblyBot Advanced | Printing the hydrogel on the half-sphere structure |
| 400 from Advanced Solutions Life Sciences (extrusion method) | Structure with a diameter approximately of 20 cm |
| BioX from CellInk (extrusion method) | Human ear (35% in size) |

## Claims

1. Composition which is a hydrogel comprising, in an aqueous medium:
- at least one hydrophilic thickening polymer, and
- particles of eggshell membrane, in an amount of less than 5% by weight of the total weight of the composition, wherein said particles are rod-shaped, needle-shaped or fibrous, having a particle size equal to or less than 50 µm.

2. Composition according to claim 1, wherein the particles of eggshell membrane are present in an amount of 0.1% to 4% by weight, preferably from 0.25% to 2% by weight of the total weight of the composition, and/or the particles of eggshell membrane have a D99.5 particle size equal to or less than 40 µm, preferably a D90 particle size equal to or less than 24 µm.

3. Composition according to claim 1 or 2, which comprises at least two hydrophilic thickening polymers.

4. Composition according to any one of claims 1 to 3, wherein the hydrophilic thickening polymer is chosen from alginates and carrageenans; polymers of animal origin, possibly modified ; anionic, cationic, amphoteric or non-ionic chitin or chitosan polymers; cellulose polymers; vinyl polymers ; polymers of natural origin, possibly modified, such as galactomannans and derivatives thereof, xanthan gum and the derivatives of xanthan, agar and its derivatives; mucopolysaccharides ; homo- or copolymers of acrylic or methacrylic acid or the salts thereof and the esters thereof; acrylic and acrylamide acid copolymers ; polyacrylic acid/alkyl acrylates copolymers ; homopolymers and copolymers with an acrylamido propane sulfonic acid base ; and mixtures thereof, preferably the hydrophilic thickening polymer is chosen from alginates and carrageenans; gelatin, collagen; anionic, cationic, amphoteric or non-ionic chitin or chitosan polymers; hydroxyethylcellulose, hydroxypropylcellulose, hydroxymethylcellulose, ethylhydroxyethylcellulose, carboxymethylcellulose, and quaternized cellulose derivatives; polyvinylpyrrolidones, methylvinyl ether and malic anhydride, vinyl acetate and crotonic acid copolymer, vinylpyrrolidone and vinyl acetate copolymers; vinylpyrrolidone and caprolactam copolymers; polyvinyl alcohol; Konjac gum, Gellan gum, Carob gum, Fenugrec gum, Karaya gum, Tragacanth gum, gum arabic, gum acacia, guar gum, hydroxypropylguar, hydroxypropylguar modified by sodium methylcarboxylate groups, ammonia trimethyl hydroxypropyl guar chloride, xanthan gum, agar and agarose; chondroitin sulfates, hyaluronic acid, sodium hyaluronate, sodium acetylated hyaluronate, dimethylsilanol hyaluronate, sodium stearoyl hyaluronate, potassium hyaluronate, propyleneglycol hyaluronate, sodium hyaluronate crosspolymer, methacrylated hyaluronic acid, hydroxypropyl trimonium hyaluronate, hydrolyzed hyaluronic acid, hydrolyzed sodium hyaluronate and zinc hydrolyzed hyaluronate; polyacrylic acids and the salts, in particular sodium, of polyacrylic acid and more particularly a cross-linked sodium polyacrylate ; sodium polymethacrylate, the sodium salts of polyhydroxycarboxylic acids ; carboxyvinyl polymers modified or not ; polyacrylamidomethyl propane sulfonic acid partially neutralized with ammonia and highly cross-linked ; copolymers of acrylamidomethyl / acrylamide propane sulfonic acid ; copolymers of acrylamidomethyl / methylacrylate propane sulfonic acid of polyoxyethylene alkyl (cross-linked or not) ; copolymers of acrylamidomethyl propane sulfonic acid and of hydroxyethyl acrylate ; and mixtures thereof; preferably the hydrophilic thickening polymers are at least alginate and gelatin.

5. Composition according to any one of claims 1 to 4, which comprises an amount of hydrophilic thickening polymer(s) comprised between 0.01 and 20 % by weight of the total weight of the composition.

6. Composition according to any one of claims 1 to 5, wherein the particles of eggshell membrane comprise at least one cysteine-rich eggshell membrane protein (CREMP) and/or collagen X; preferably comprise CREMPs, collagen X, lysyl oxidase-like protein 2 and lysozyme; and more preferably CREMPs and collagen X represent at least 40% by weight of the total weight of the particles, and/or wherein the composition comprises at least one additive chosen from growth factors and differentiation factors.

7. A bioink comprising a composition according to any one of claims 1 to 6 and isolated cells.

8. Kit comprising:
- a first composition, wherein said first composition is according to any one of claims 1 to 6 or a bioink according to claim 7; and
- a second composition which comprises, in a compatible medium, at least one crosslinking agent; and
- optionally a third composition comprising at least one compound chosen from cells, coloring agents, pharmacologic agents, differentiation factors, growth factors, biological markers and their mixtures.

9. Kit according to claim 8, wherein said crosslinking agent is chosen from:
- polyvalent metal salts, preferably chosen from calcium, iron, silver, strontium, aluminum, manganese, selenium, copper and zinc, and
- chemical crosslinking agents, preferably chosen from carbodiimides, particularly water-soluble carbodiimides such as 1-ethyl-3-(3-dimethylaminopropyl)carbodiimide (EDC), N-cyclohexyl-N'-(2-morpholinoethyl)carbodiimide (CMC), dicyclohexylcarbodiimide (DCC), N-hydroxysuccinimide (NHS) and CDI (carbodiimidazole).

10. Use of a composition according to any one of claims 1 to 6, or of a bioink according to claim 7, or of a kit according to claim 8 or 9, as an *in vitro* research model, or for obtaining a 3D organoid, or for obtaining a 2D product or an isolated 3D organ or tissue.

11. Method for manufacturing a 2D or a 3D product, comprising the following steps :
(a) preparing a composition according to any one of claims 1 to 6 or a bioink according to claim 7 for bioprinting, preferably by heating said composition to a temperature comprised between 35°C and 40°C and centrifugating it;
(b) introducing the composition obtained at the end of step (a) into at least one first cartridge of a bioprinter, said bioprinter comprising at least one printbed, at least one printhead linked to said first cartridge and having a nozzle, preferably two printheads each having a nozzle, and optionally a UV curing system; and
(c) manufacturing the 2D or 3D product in the bioprinter,
preferably wherein step (c) is performed at a printhead temperature comprised between 35°C and 40°C, a printbed temperature comprised between 3°C and 12°C, a printing pressure comprised between 25 and 35 KPa, a nozzle speed comprised between 5 and 15 mm/s, and a nozzle inner diameter comprised between 0.23 to 0.45 mm.

12. Method according to claim 11, wherein the 2D or 3D product obtained at the end of step (c) is mixed with at least one crosslinking agent.

13. Method according to claim 11 or 12, wherein :
- cells are added during step (a) or the composition or bioink already comprises cells, or
- cells are introduced into at least one second cartridge of a bioprinter, said bioprinter comprising two printheads, a first printhead being linked to the first cartridge and a second printhead being linked to said second cartridge, and cells are bioprinted during step (c) or after step (c), or
- cells are added after step (c) once the 2D or 3D product is obtained.

14. Method according to any one of claims 11 to 13, wherein the 2D or 3D product is an organ, a tissue or an organoid.

15. Monolayer 2D product or isolated 3D organ or tissue which is obtainable by a method according to any one of claims 11 to 14.
